# FASCICULE DE BREVET EUROPEEN

(11) **EP 1 450 696 B1**
(45) Date de publication et mention de la délivrance du brevet: **26.08.2009**
(21) Numéro de dépôt: 02795361.1
(22) Date de dépôt: 04.11.2002
(51) Int. Cl.: A61B 17/15, A61B 19/00

(54) **Dispositif pour la mise en oeuvre d'un procédé de sélection d'éléments de prothèse de genou**
Vorrichtung zum Durchführen eines Verfahrens zur Auswahl von Knieprothesenelementen
Device for carrying out a method for selecting knee prosthesis elements

(30) Priorité: 05.11.2001 FR 0114287
(43) Date de publication de la demande: 01.09.2004
(73) Titulaire: Depuy (Ireland) Limited, Ringaskiddy, County Cork (IE); Lefevre, Christian, 29200 Brest (FR); Stindel, Eric, 29280 Locmania Plouzane (FR); Briard, Jean-Louis, 76130 Mont Saint Aignan (FR); Merloz, Philippe, 38330 Saint Imier (FR)
(72) Inventeur: LEFEVRE, Christian, F-29200 Brest (FR); STINDEL, Eric, F-29280 Locmania Plouzane (FR); BRIARD, Jean-Louis, F-76130 Mont Saint Aignan (FR); MERLOZ, Philippe, F-38330 Saint Imier (FR)
(74) Mandataire: Bernasconi, Jean Raymond
(86) Numéro de dépôt international: PCT/FR2002/003770
(87) Numéro de publication internationale: WO 2003/039377

(56) Documents cités:
- WO-A-99/23956
- WO-A-99/60939
- US-A- 5 682 886
- US-A- 5 824 085
- US-A- 6 002 859
- KUNZ M. ET AL: 'A non-CT based total knee arthroplasty system featuring complete soft-tissue balancing' LECTURE NOTES IN COMPUTER SCIENCE vol. 2208, 14 Octobre 2001 - 17 Octobre 2001, pages 409 - 415

## Description

La présente invention a trait à un dispositif pour la mise en oeuvre d'un procédé de sélection d'une prothèse de genou, et plus précisément un procédé de sélection qui permet de choisir un ou des éléments d'une prothèse de genou, tels que, notamment implant fémoral et tibial de prothèse et/ou cale tibiale ou fémorale, à l'intérieur d'un jeu disponible d'éléments.

Le procédé mis en oeuvre à l'aide du dispositif peut également permettre de déterminer, sur un modèle informatique du genou du patient, des plans de résection, notamment de résection fémorale et tibiale, destinées à servir d'assise à la partie correspondante de prothèse de genou.

Dans les opérations classiques de pose de prothèse de genou, le chirurgien procède aux coupes osseuses tibiales et fémorales en fonction des caractéristiques anatomiques du patient et du type de prothèse disponible commercialement, puis procède, en cours de pose, à des réglages, par exemple en utilisant des cales, ou même en refaisant une coupe de résection, afin d'optimiser autant que possible les propriétés articulaires de la prothèse lorsqu'elle sera en fonctionnement.

On conçoit que cette optimisation dépend fortement du savoir-faire du chirurgien et des particularités anatomiques du genou à opérer.

L'objectif recherché est d'obtenir, si possible, des tensions égales des parties molles du genou à 0 et 90° qui se maintiennent sur l'ensemble de l'arc de flexion de la prothèse, un alignement géométrique satisfaisant et une extension sans flexum, pour optimiser les contraintes à la station debout, et obtenir la meilleure adéquation possible avec l'anatomie du patient. Un objectif important est l'obtention d'une bonne stabilité du genou par un équilibrage ligamentaire approprié.

Dans ce but, on a déjà proposé d'assister le chirurgien par des moyens de mesures et de traitement des données de mesures par ordinateur.

De tels moyens et méthodes sont décrits par exemple dans KUNTZ M et Al. « A Non-CT Based Total Knee Arthroplasty System Featuring Complete Soft - Tissue Balancing » - Lecture notes in computer Science, Vol. 2208, Actes de la conference MICCA/ 2001, pages 409 - 415 - Springer - Verlag, 2001, sur lequel se base le préambule de la revendication 1 ammenée, ainsi que dans US-A-5 682 886 et WO 99/60939.

Ainsi, il est connu de mémoriser la conformation anatomique des extrémités distale du fémur du patient et proximale du tibia à partir de données de mesures obtenues par un moyen quelconque. Celles-ci peuvent être obtenues, par exemple, par scannerisation, ou de préférence, par mesure in situ. On peut utiliser dans un système de référence spatial tridimensionnel, défini à l'aide de marqueurs référence (par exemple, billes de référence réfléchissantes), les rayons infra-rouges ou magnétiques fixés en trois positions convenables sur une épiphyse du genou, par déplacement d'un palpeur également repéré dans l'espace par le moyen d'acquisition, un logiciel de type connu permettant de reconstituer la forme tridimensionnelle précise des extrémités en question. Un tel dispositif comporte, plus précisément, un émetteur-récepteur de localisation, tel qu'une caméra haute-définition infra-rouge, permettant le repérage des points de référence fixés sur le patient et des instruments marqués utilisés, tels que pointeur ou palpeur, guide de coupe, etc., des moyens de mémorisation et de calcul, tels qu'un ordinateur dans lequel est mis en oeuvre un logiciel de modélisation du type 3D, de préférence un moyen de visualisation tel qu'un écran, et un moyen de commande tel que souris ou écran tactile ou de préférence pédale actionnée au pied par le chirurgien.

Un marquage convenable sur la partie de genou mobile par rapport au système de référence permet également de calculer les positions relatives du fémur et du tibia.

Il est ainsi connu, après résection du plateau tibial, et éventuellement mise en place d'un élément de support de plateau prothétique tibial, et en utilisant un tenseur introduit par le chirurgien dans l'espace entre l'extrémité tibiale et l'extrémité articulaire fémorale, de déterminer, sous la valeur de tension choisie imposée par le tenseur, l'écartement entre le tibia et le fémur ainsi que l'angle HKA, c'est à dire angle, pris en interne, entre l'axe mécanique fémoral (défini par le centre de la hanche et le centre du genou) et l'axe mécanique tibial (défini par le centre du genou et le centre de la cheville), d'une part, en extension, ou dans une position aussi proche que possible de l'extension, d'autre part, en flexion à 90°, le chirurgien procédant ensuite au choix des éléments constitutifs de prothèse les mieux adaptés dans le jeu d'éléments disponibles, ce choix pouvant être visualisé sur l'écran, avant la pose, par la modélisation de la position de l'élément présélectionné dont les caractéristiques ont été mises en mémoire dans l'ordinateur.

On constate cependant que cette technique n'assure pas toujours un choix et/ou positionnement optimal du ou des éléments de prothèse sélectionnés, ce qui ne permet pas d'obtenir une biomécanique optimale, notamment lors de la rétraction des parties molles postérieures du genou en flexum, et dans les phases de flexion intermédiaires entre 0 et 90° ainsi qu'au delà de 100°. La biomécanique optimale correspond à une « bonne tension » des parties molles dans tout le secteur de mouvement, à savoir tension de stabilité pour les zones d'appui et microjeu de laxité entre 20 et 140°, permettant une mobilité facile sans jamais d'hypertension ni de laxités inégales ou exagérées.

La présente invention se propose de remédier à ces inconvénients et d'améliorer les possibilités de sélectionner le ou les éléments prothétiques, afin d'assurer le meilleur fonctionnement de la prothèse de genou dans toutes les positions naturelles d'extension et de flexion et notamment, en assurant une laxité qui soit, à la fois d'une valeur convenable et qui reste sensiblement constante dans tout le domaine de mobilité de la prothèse.

Un autre objectif est de déterminer, sur le modèle informatique de genou, les positions optimales des plans de coupe pour la résection de l'extrémité fémorale distale et/ou tibiale proximale.

L'invention a pour objet un dispositif pour la mise en oeuvre d'un procédé de sélection d'un ou de plusieurs éléments de prothèse de genou, et notamment d'une partie fémorale de prothèse, et/ou de plateau prothétique tibial à l'intérieur d'un jeu disponible de ces éléments, et/ou d'un gabarit d'épaisseur ou cale fémoral ou tibial, caractérisé en ce que l'on acquiert les informations spatiales d'écartement et de position fémoro-tibials sous tension, rotule en place ou luxée, y compris l'angle HKA correspondant, pour au moins trois positions angulaires du genou, à savoir une position intermédiaire de l'ordre de 20° en flexion, une position en extension, si possible à 0° de flexion, et une position de flexion importante, de préférence de l'ordre de 90° en flexion, l'on traite les informations ainsi obtenues correspondant à la susdite position en flexion réduite de 20° et la position en extension de façon à indiquer si, dans ces deux positions, les angles HKA sont sensiblement égaux et compris dans les limites tolérables en cas de genu varum et en cas de genu valgum, et que l'on traite ces informations, notamment celles correspondant à l'extension et à la flexion importante de l'ordre de 90°, pour déterminer les tailles et/ou les positions d'implant, et notamment le choix de l'épaisseur de l'insert tibial pour combler, au mieux, l'espace entre le condyle prothétique et la coupe tibiale, et le choix de la taille de l'implant fémoral.

Par angle de flexion intermédiaire, on entend un angle de l'ordre de 20° ± 10° et de préférence de 20° ± 5° ; cet angle est dénommé ci-dessous 20°.

L'angle en extension est, de préférence, de 0° ± 10°, notamment 0° ± 5° ; cet angle est dénommé ci-dessous 0°. L'angle de grande flexion, de préférence de 90°, peut être de 90° ± 15°, notamment 90° ± 10°, de préférence 90° ± 5° ; cet angle est dénommé ci-dessous 90°.

De préférence, les informations sont acquises dans l'ordre suivant : informations à 20°, puis informations à 0°, puis informations à 90°.

Selon ce procédé, on vérifie, grâce aux moyens de l'ordinateur, que les angles auxquels les données sont acquises, correspondent bien aux angles précités, l'acquisition étant refusée si l'angle correspondant n'est pas contrôlé.

L'acquisition et le traitement des informations correspondant à l'angle intermédiaire de 20° et à l'angle d'extension à 0° pourront être utilisés par le chirurgien soit pour considérer que les propriétés anatomiques du genou sont convenables, soit pour réduire un éventuel flexum (impossibilité d'obtenir une extension complète) ou réaliser une libération convenable des ligaments collatéraux en cas de valgum ou de varum exagéré.

De préférence, selon le procédé on détermine si, aux angles d'extension et de flexion à 20°, l'angle HKA est sensiblement égal dans les deux positions et compris entre 175° et 180° en cas de genu varum et entre 180 et 184° en cas de genu valgum, auquel cas le procédé considère que les propriétés anatomiques sont convenables.

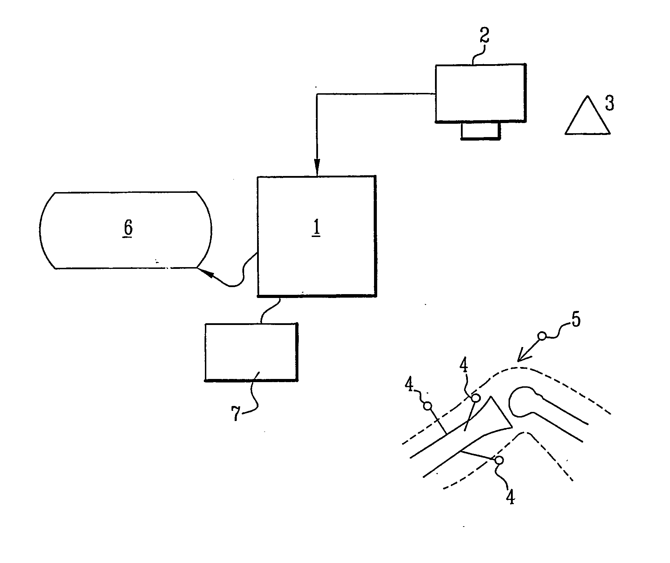

Il est également prévu dans le cadre de l'utilisation du procédé mis en oeuvre grâce à l'invention, des acquisitions d'informations relatives, notamment, à l'écartement fémoro-tibial et à l'angle HKA, pour d'autres positions intermédiaires de flexion, notamment 45°, ou même en continu. Le contrôle continu de la tension des parties molles fémur par rapport à tibia, associé à la mesure de l'angle HKA entre 0 et 140°, permet d'obtenir un alignement fémoro-tibial correct ; surtout il permet de calculer le positionnement idéal des implants. En effet, la cinétique fémur/tibia comporte notamment un mouvement de flexion-extension dont il est important de connaître les centres de rotation. Ceux-ci peuvent être calculés successivement entre 0 et 140° grâce à l'invention. La connaissance de ces centres de rotation dits « anatomiques » permettra de mieux déterminer la position des centres de rotation dits prothétiques en rapport avec le dessin de la prothèse. Mais dans le cadre de l'invention, les informations recueillies comprennent au moins celles qui correspondent aux angles de 0° (ou extension), flexion réduite et flexion importante, et les moyens de traitement mis en oeuvre dans le procédé vérifient que les mesures prises correspondent bien à ces angles, tels que restitués par les moyens de traitement mis en oeuvre dans le procédé.

De préférence, le traitement relatif à la détermination des éléments les plus appropriés de la prothèse du procédé n'est poursuivi qu'une fois que les informations correspondant à 0° et 20° sont acceptables, à savoir absence de flexum sensible et angles HKA sensiblement égaux et dans les valeurs admises. Une fois ces vérifications faites, le traitement informatique modèlise l'articulation en y incorporant les informations dimensionnelles relatives aux différents composants des jeux de prothèses préalablement mémorisés pour former des modèles de genou avec prothèses en position implantée.

Cette modélisation assure que la position antéro-postérieure de la prothèse fémorale est telle que :
le bord supérieur de la trochlée prothétique est en contact avec la corticale fémorale antérieure ou légèrement postérieur dans le cas où la taille réelle du fémur du patient tombe entre deux tailles prothétiques ;
la rotation axiale de la prothèse fémorale est telle que les condyles prothétiques postérieurs sont parallèles à la coupe tibiale réelle,
l'insert tibial choisi dans le jeu comble au mieux l'espace entre les condyles prothétiques et la coupe tibiale ;
la position medio-latérale est telle que l'implant est centré sur l'échancrure anatomique,
et la position distale de l'implant fémoral est choisie en utilisant le plateau tibial sélectionné et la position d'extension mémorisée pour qu'en position d'extension, l'implant fémoral soit bien en contact avec l'insert tibial déterminé en flexion sans laxité résiduelle;
le varus fémoral est tel pour qu'en extension les condyles prothétiques distaux sont parallèles à la coupe tibiale réalisée ;
le flexum fémoral est sensiblement nul.

Cette optimisation par modélisation peut être entièrement automatique, le procédé indiquant alors au chirurgien le choix exact des différents éléments du jeu prothétique. Grâce à l'enregistrement des positions des coupes distales fémorales et proximales tibiales entre 0 et 140°, après choix des coupes et des implants - on pourra simuler sur écran - le fonctionnement du genou entre 0 et 140°.

Cette étape permet d'éliminer toute hypertension ou laxité excessive entre 0 et 140°. Un logiciel permet de définir la position optimale des implants fémur et tibia et donc des coupes pour s'harmoniser ou mieux avec l'anatomie et les parties molles.

Dans un autre mode de mise en oeuvre, le procédé permet au chirurgien de présélectionner des paramètres inhérents au jeu, tels que la taille de l'implant fémoral, la hauteur de l'insert tibial, la rotation axiale, le varus fémoral, le flexum, la position antéro-postérieure, la position latérale, la hauteur de la coupe fémorale distale à effectuer.

De façon avantageuse, les laxités interne et externe estimées en flexion et en extension sont affichées en continu, au moins pour les trois positions précitées et de préférence sur toute position de flexion modélisée.

Le dispositif selon l'invention destiné à la mise en oeuvre le procédé décrit ci-dessus, comporte: un moyen d'acquisition de la position spatiale d'un système de repères de référence spatial tridimensionnel sur le tibia ou le fémur d'un patient,et des positions spatiales de repères, palpeurs ou instruments sur l'autre os pour obtenir des informations spatiales relatives au déplacement entre le fémur et le tibia d'un patient au niveau du genou, des moyens, comportant de préférence des données anatomiques du fémur et/ou du tibia du patient obtenues préalablement, pour pouvoir déterminer, en fonction desdites informations spatiales, l'angle de flexion entre le fémur et le tibia, l'écartement entre les extrémités du fémur et du tibia ainsi que l'angle HKA, des moyens de traitement et de mémorisation pour mémoriser ledit écartement et ledit angle HKA, en association avec l'angle de flexion, pour au moins trois angles de flexion, à savoir un ange de flexion réduite, notamment de 20° défini ci-dessus, un angle d'extension, notamment un angle de 0° tel que défini ci-dessus, et un angle de flexion importante, notamment un angle de 90° tel que défini ci-dessus, des moyens pour comparer lesdits écartements et angles HKA, au moins pour l'angle de flexion réduite et l'angle d'extension, des moyens de mémorisation comprenant des informations dimensionnelles relatives à des jeux de composants d'implants de genou, des moyens de traitement 3D pour modéliser des positions implantées desdits éléments de jeu de prothèse d'implant, au moins dans les positions d'angles d'extension et de grande flexion, et des moyens de traitement pour fournir des informations pertinentes relatives à l'implant de genou modélisé et/ou de sélection desdits éléments de jeu d'implant procurant les meilleures informations caractéristiques, des moyens de visualisation desdites informations ou sélection, et des moyens de commande pour le fonctionnement du dispositif.

Lesdits moyens de commande sont de préférence conçus pour permettre à l'opérateur d'enregistrer les données captées correspondant au moins auxdits angles de faible flexion, d'extension et de forte flexion en association avec les moyens de traitement vérifiant et validant la bonne valeur de l'angle pour l'enregistrement.

De préférence, lesdits moyens de commande permettent également à l'opérateur de choisir un ou plusieurs éléments du jeu d'éléments de prothèse de façon à permettre auxdits moyens de traitement de modéliser l'implantation desdits éléments et de fournir ou traiter les caractéristiques pertinentes pour lesdits éléments en position de modélisation implantée.

De préférence, les moyens de commande comportent des moyens actionnables au pied ou à la voix ou conçus d'une autre façon pour permettre à un chirurgien de commander le dispositif sans avoir à utiliser ses mains.

Les moyens d'acquisition permettant de capter les informations spatiales relatives aux déplacements et aux positions relatives du fémur et du tibia comportent, de préférence, une caméra de type numérique haute définition sensible à des signaux provenant des parties osseuses du genou, tels que, par exemple, des marqueurs à réflexion infra-rouge ou, éventuellement, des moyens de reconnaissance d'image.

Les moyens de modélisation 3D peuvent être des logiciels de modélisation du commerce, adaptés, le cas échéant, à la modélisation des parties osseuses.

Les moyens de traitement mettent en oeuvre les acquisitions et traitements suivants :
- acquisition des repères anatomiques nécessaires à l'axe mécanique (centre hanche et cheville),
- acquisition de points anatomiques divers au niveau du genou,
- acquisition et modélisation de la surface tibiale,
- planification tibiale,
- positionnement du guide, fixation et coupe tibiale,
- vérification de la planéité de la coupe tibiale,
- tension et acquisition à 20°,
- tension et acquisition à 0°,
- comparaison des 2 valeurs et décision,
- tension et acquisition à 90°,
- planification fémorale,
- positionnement des guides de coupe fémoraux, fixation et coupes.

L'invention est particulièrement adaptée pour mettre en oeuvre un procédé de pose de prothèse articulaire du genou comprenant la succession des gestes suivantes :
- acquisition usuelle des repères anatomiques nécessaires à l'axe mécanique, notamment centre hanche et cheville,
- pose de marqueurs de référence sur des parties osseuses de genou et acquisition usuelle de ces points anatomiques,
- acquisition et modélisation de la surface tibiale,
- planification tibiale, à savoir détermination de l'implant tibial et de sa position,
- positionnement et fixation d'un guide de coupe tibial et résection par coupe du plateau tibial, éventuellement pose d'une base de plateau tibial prothétique,
- vérification de la planéité de la coupe tibiale,
- distraction par tenseur de l'intervalle fémoro-tibial dans au moins deux positions angulaires, à savoir une position de flexion de 20° tel que défini ci-dessus, une position d'extension,
- obtention des écartements osseux et de l'angle HKA pour les deux positions par le dispositif selon l'invention,
- si nécessaire, libération postérieure en cas de flexum et/ou libération de ligaments collatéraux, jusqu'à obtention des angles HKA convenables dans lesdites positions, çette vérification étant effectuée par le dispositif selon l'invention,
- distraction à flexion élevées, notamment de 90° sous tenseur et obtention des écartements osseux et angle HKA correspondants,
- planification fémorale incluant :

- présélection manuelle de différents éléments d'implant en fonction des résultats desdites mesures et vérification des valeurs obtenues par modélisation,
- et/ou obtention d'une sélection automatique desdits éléments avec indication desdites caractéristiques pertinentes, et
- positionnement des guides de coupe fémoraux, fixation, coupes et implantation du ou des éléments sélectionnés.

D'autres avantages et caractéristiques de l'invention apparaîtront à la lecture de la description suivante, faite à titre d'exemple non limitatif, et se référant au dessin annexé dans lequel :
- la figure unique représente une vue schématique d'un dispositif selon l'invention.

Le dispositif selon l'invention comporte un ordinateur 1 pour la mise en oeuvre du procédé
- Il comprend également une caméra numérique haute définition 2 associée à une source d'émission infra-rouge 3 couvrant le champ dans lequel évoluent un ou des ensembles de trois marqueurs 4 qui renvoient, de façon passive, le rayonnement infra-rouge. D'une façon en soi connue, un groupe de trois marqueurs est mis en place par le chirurgien sur un membre ou sur un os, par exemple sur la partie inférieure du fémur pour former un système de marquage tri-dimensionnel permettant à l'ensemble caméra-ordinateur de déterminer de façon classique la localisation géométrique exacte d'un ou de plusieurs marqueurs supplémentaires 5, par exemple placés sur une partie osseuse mobile par rapport au système de référence des trois marqueurs. De tels dispositifs sont bien connus dans le domaine de l'analyse des formes et de la modélisation informatique et n'ont pas besoin d'être décrits davantage en détail ici, les logiciels de modélisation étant d'ailleurs disponibles sur le marché. Un tel dispositif permet, par exemple, à un chirurgien qui déplace de façon convenable le marqueur mobile sur une surface anatomique, de reproduire par modélisation cette surface.

Le logiciel de modélisation mis en oeuvre dans la présente invention est conçu d'une façon en soi connue pour pouvoir calculer, par rapport au système de référence spatial des trois capteurs mis en place sur l'un des os de l'articulation, la position exacte de l'autre os de l'articulation, et notamment l'angle de flexion entre les deux os et donc les deux parties du membre inférieur, l'écartement entre les deux os, les déplacements latéraux et antéro-postérieurs, et les rotations relatives.

Le dispositif comporte encore un écran 6 susceptible d'afficher les résultats du traitement des données spatiales par l'ordinateur ainsi que les différents autres éléments pertinents du logiciel de façon à pouvoir être vus par le chirurgien, ainsi qu'un dispositif de commande 7 qui peut être, par exemple, un clavier classique d'ordinateur, ou l'écran 6 constitué en tant qu'écran tactile, ou encore, de préférence, un dispositif de commande à pédale permettant d'être mis en oeuvre par le pied du chirurgien.

L'utilisation du dispositif selon l'invention s'effectue de la façon suivante.

Le chirurgien dispose d'un jeu stérile d'implants de différentes tailles, chaque implant comportant, classiquement : un composant tibial formé d'une embase coopérant avec une tige tibiale pour le bon scellement de l'embase sur la surface de coupe du plateau tibial, avec pour chaque type d'embase, un jeu de plateau tibiaux, par exemple en polyéthylène susceptible d'être rapportés sur l'embase pour fournir la surface articulaire prothétique tibiale ; un composant fémoral comportant une extrémité distale coopérant avec une tige fémorale destinée au scellement dans le canal médulaire fémoral, avec une pièce de trochlée prothétique destinée à être articulée avec le plateau tibial, cette pièce étant soit directement solidaire de l'extrémité distale fémorale, soit, dans d'autres modèles, susceptible d'y être rapportée par exemple avec interposition de cales d'un jeu de cales d'épaisseurs variables ; le composant tibial et le composant fémoral étant, en outre, réunis de façon articulaire par un moyen de pivotement.

Après avoir positionné les différents marqueurs infra-rouge, puis assuré l'acquisition des formes anatomiques des parties pertinentes du fémur et du tibia et obtenu grâce à l'ordinateur la modélisation anatomique exacte de ces formes et dimensions (en variante ces données dimensionnelles peuvent déjà avoir été introduites dans l'ordinateur préalablement, par exemple par scannerisation pré-opératoire), le chirurgien pratique la résection du plateau tibial délabré et, à l'aide du marqueur mobile, marque la position de ce plan de coupe qui est incorporé dans le modèle informatique.

De préférence, pendant l'ensemble de l'opération, l'écran de l'ordinateur présente en continu la valeur de la flexion courante sur une vue latérale du genou. L'angle HKA est également affiché en permanence sur une vue frontale.

Le chirurgien procède ensuite aux étapes suivantes après la résection tibiale :

Le chirurgien met le genou en flexion de 20°. Lorsque la flexion est d'environ 20° (tolérance de ± 5°), le chirurgien insère le tenseur et met le genou en tension satisfaisante.

Il actionne les moyens de commande 7 et l'ensemble caméra-ordinateur acquiert et mémorise la totalité des données de la position relative entre le fémur et le tibia à cet angle.

Dans un deuxième temps, le chirurgien amène le genou aux alentours de 0° de flexion, c'est à dire en extension. Lorsque cette valeur est atteinte (avec une tolérances de ± 5°), le chirurgien insère le tenseur et met le genou en extension satisfaisante.

Il actionne à nouveau les moyens de commande 7 et l'ensemble caméra-ordinateur acquiert et mémorise la totalité des données de la position relative du fémur et du tibia.

A chaque actionnement l'ensemble caméra-ordinateur vérifie que l'angle de flexion est bien de 20° ± 5° puis de 0° ± 5°. Les données relatives auxdites positions, à savoir la laxité ou plus précisément les laxités interne et externe au niveau des condyles interne, respectivement externe et l'angle HKA sont mémorisées et associées à l'angle correspondant.

Le logiciel compare alors la valeur de l'angle HKA à 20° et la valeur de l'angle HKA à 0°. Il vérifie également que cet angle est compris entre 175 et 180° en cas de genu varum et entre 180 et 184° en cas de genu valgum.

Si ces deux conditions sont satisfaites et qu'il n'existe pas de problème de flexum, le chirurgien poursuit. On passe alors à l'étape suivante en mettant le genou en flexion aux alentours de 90° de flexion.

Si au contraire, l'une de ces conditions n'est pas satisfaite, il pratique une libération postérieure en cas de flexum, et recommence les mesures autour de 20° puis autour 0°, jusqu'à ce que les deux étapes fournissent des valeurs HKA sensiblement identiques ; si ceci n'est pas le cas, il procède à une libération des ligaments collatéraux de façon classique jusqu'à l'obtention du résultat convenable.

Lorsque ces critères sont satisfaits, et après avoir mis le genou à 90°, il insère à nouveau le tenseur et met le genou en tension satisfaisante.

Les positions relatives du tibia et du fémur sont également mémorisées à l'angle de 90° par action du chirurgien sur le moyen de commande.

Une fois ces différentes acquisitions faites, l'ordinateur est en position de proposer automatiquement la position de l'implant fémoral et son orientation en rotation, ainsi que la hauteur de l'insert tibial à choisir, en tenant compte des critères et contraintes suivants :
- la position de la trochlée fémorale prothétique dans le plan antéro-postérieur est obtenue par contact du bord supérieur de la trochlée prothétique avec la corticale fémorale antérieure ;
- la rotation axiale (dans le fût fémoral) du composant fémoral est telle que les condyles prothétiques postérieurs sont parallèles à la coupe tibiale qui a été réalisée ;
- L'épaisseur de l'insert tibial (embase + plateau tibial) est choisie pour combler au mieux l'espace entre les condyles prothétiques et la coupe tibiale sans être supérieur à cet espace. Dans le cas où l'insert tibial le moins épais est tout de même supérieur à l'espace disponible, le logiciel permettra d'obtenir la position de la recoupe tibiale théorique à réaliser pour pouvoir placer cet insert ;
- la position medio-latérale est telle que l'implant est centré sur l'échancrure anatomique entre les condyles anatomiques ;
- la position distale de l'implant est calculée en utilisant le plateau tibial choisi et la position en extension équilibrée qui a été mémorisée auparavant. La hauteur de coupe distale est telle qu'en position d'extension l'implant fémoral est en contact avec l'insert tibial déterminé en flexion ?. Il s'agit d'une contrainte que l'on se donne sur la laxité en extension.

Le varus fémoral est tel qu'en extension les condyles prothétiques distaux sont parallèles à la coupe tibiale réalisée.

Le flexum fémoral est nul, l'angle de coupe fémoral par rapport à la perpendiculaire à l'axe mécanique fémoral de profil étant déterminé en fonction du type de prothèse, certains types prévoyant un angle non nul, par exemple, 15°.

Pour chaque position l'écran indique l'angle de flexion, les laxités interne et externe et l'angle HKA.

La proposition qui est ainsi faite par le dispositif selon l'invention peut être modifiée en actionnant les moyens de commande 7, par exemple pour modifier les paramètres suivants :
- taille de l'implant fémoral (en général six tailles sont disponibles)
- hauteur de l'insert (en général de 10 à 20 mm),
- rotation axiale,
- varus fémoral,
- flexum,
- position antéro-postérieure,
- position latérale,
- hauteur de la coupe distale fémorale.

A chaque modification volontaire ainsi réalisée, les valeurs de laxité sont recalculées en utilisant les positions mémorisées à 0° et 90°.

Enfin, s'il n'a pas été possible d'obtenir des laxités satisfaisantes en flexion, le chirurgien peut :
- choisir l'implant de taille inférieure et le déplacer en postérieur en empiétant sur la corticale fémorale antérieure,
- choisir l'implant de taille inférieure, conserver la position antéro-postérieure de l'implant et accepter la laxité.
- choisir l'implant de taille supérieure et accepter de diminuer la laxité si elle est trop importante,
- libérer les ligaments ou parfaire la surpression des ostéophytes.

## Revendications

1. Dispositif destiné à la mise en oeuvre d'un procédé de sélection d'un ou de plusieurs éléments de prothèse de genou, et notamment d'une partie fémorale de prothèse, et/ou de plateau prothétique tibial à l'intérieur d'un jeu disponible de ces éléments, et/ou d'un gabarit d'épaisseur fémoral ou tibial, ledit dispositif comportant : un moyen (2, 3) d'acquisition de la position spatiale d'un système de repères (4, 5) de référence spatial tridimensionnel sur le tibia ou le fémur d'un patient, et des positions spatiales de repères, palpeurs ou instruments sur l'autre os pour obtenir des informations spatiales relatives au déplacement entre le fémur et le tibia d'un patient au niveau du genou, des moyens, comportant de préférence des données anatomiques du fémur et/ou du tibia du patient obtenues préalablement, pour pouvoir déterminer, en fonction desdites informations spatiales, l'angle de flexion entre le fémur et le tibia, l'écartement entre les extrémités du fémur et du tibia ainsi que l'angle HKA, c'est-à-dire l'angle pris en interne entre l'axe mécanique fémoral défini par le centre de la hanche et le centre du genou et l'axe mécanique tibial défini par le centre du genou et le centre de la cheville, des moyens (1) de traitement et de mémorisation pour mémoriser ledit écartement et ledit angle HKA, en association avec l'angle de flexion, pour un angle d'extension, notamment un angle de 0° ±5°, et un angle de flexion importante de l'ordre de 90°, notamment un angle de 90° ± 15°, des moyens de mémorisation comprenant des informations dimensionnelles relatives à des jeux de composants d'implants de genou, des moyens de traitement 3D pour modéliser des positions implantées desdits éléments de jeu de prothèse d'implant, au moins dans les positions d'angles d'extension et de grande flexion, et des moyens de traitement pour fournir des informations pertinentes relatives à l'implant de genou modélisé et/ou de sélection desdits éléments de jeu d'implant procurant les meilleures informations caractéristiques, des moyens de visualisation (6) desdites informations ou sélection, et des moyens de commande (7) pour le fonctionnement du dispositif, **caractérisé en ce que** les moyens des traitements et de mémorisation sont adaptés pour mémoriser en outre ledit écartement et ledit angle HKA, pour au moins un angle de flexion réduite, notamment un angle de 20° + 10°, et **en ce qu'**il comprend des moyens pour comparer lesdites mesures des angles HKA au moins pour l'angle de flexion réduite et pour l'angle d'extension, adaptés pour indiquer si, dans ces deux positions, les angles HKA sont sensiblement égaux et compris entre des limites tolérables en cas de genu varum et en cas de genu valgum.

2. Dispositif selon la revendication 1 **caractérisé en ce que** lesdits moyens de commande (7) sont conçus pour permettre à l'opérateur d'enregistrer les données captées correspondant au moins auxdits angles de faible flexion, d'extension et de forte flexion, lesdits moyens de traitement vérifiant et validant que les angles auxquels les données sont acquises correspondant bien aux angles précités.

3. Dispositif selon l'une des revendications 1 et 2 **caractérisé en ce que** lesdits moyens de commande (7) sont conçus pour proposer à l'opérateur de choisir un ou plusieurs éléments du jeu d'éléments de prothèse de façon à permettre auxdits moyens de traitement (1) de modéliser l'implantation desdits éléments et de fournir ou traiter les caractéristiques pertinents pour lesdits éléments en position de modélisation implantée.

4. Dispositif selon l'une des revendications 1 à 3
**caractérisé en ce que** les moyens de commande (7) comportent des moyens actionnables au pied ou à la voix pour permettre à un chirurgien de commander le dispositif sans avoir à utiliser ses mains.

5. Dispositif selon l'une des revendications 1 à 4 **caractérisé en ce que** les moyens d'acquisition permettant de capter les informations spatiales relatives aux déplacements et aux positions relatives du fémur et du tibia comportent une caméra de type numérique haute définition (2) sensible à des signaux provenant des parties osseuses du genou, tels que, par exemple, des marqueurs à réflexion infra-rouge (4, 5) ou, éventuellement, des moyens de reconnaissance d'image.

## Claims

1. Device which is intended for carrying out a method for selecting one or more knee prosthesis elements, and in particular a femoral prosthesis component, and/or tibial prosthetic plate from an available set of these elements, and/or a gauge for femoral or tibial thickness, the device comprising: a means (2, 3) for acquiring the spatial position of a reference system (4, 5) for three-dimensional spatial reference on the tibia or femur of a patient, and spatial reference positions, sensors or instruments on the other bone in order to obtain spatial data relating to the movement between the femur and the tibia of a patient in the region of the knee, means, preferably comprising anatomical data of the femur and/or the tibia of the patient obtained beforehand, in order to be able to determine, in accordance with the spatial data, the angle of flexion between the femur and the tibia, the spacing between the ends of the femur and the tibia and the angle HKA, that is to say, the angle taken internally between the mechanical femoral axis defined by the centre of the hip and the centre of the knee and the mechanical tibial axis defined by the centre of the knee and the centre of the ankle, processing and storage means (1) in order to store the spacing and the angle HKA in association with the flexion angle, for an angle of extension, in particular an angle of 0° ± 5°, and a significant angle of flexion in the order of 90°, in particular an angle of 90 ± 15°, storage means comprising dimensional data relating to sets of knee implant components, 3D processing means in order to model the implanted positions of the elements of the implant prosthesis set, at least in the positions of angles of extension and significant flexion, and processing means in order to provide relevant data relating to the modelled knee implant and/or in order to select the elements of the implant set which provide the best characteristic data, means (6) for displaying the data or selection and control means (7) for the operation of the device, **characterised in that** the processing and storage means are suitable for additionally storing the spacing and the angle HKA, for at least one reduced angle of flexion, in particular an angle of 20° ± 10°, and **in that** it comprises means for comparing the measurements of the HKA angles at least for the reduced angle of flexion and for the angle of extension which are capable of indicating whether, in these two positions, the HKA angles are substantially equal and between tolerable limits in the case of genu varum and in the case of genu valgum.

2. Device according to claim 1, **characterised in that** the control means (7) are configured to allow the operator to record the captured data corresponding at least to the angles of low flexion, extension and high flexion, the processing means verifying and validating that the angles at which the data are acquired correspond to the above-mentioned angles.

3. Device according to either claim 1 or claim 2, **characterised in that** the control means (7) are configured to suggest to the operator to select one or more elements from the set of prosthesis elements in order to allow the processing means (1) to model the implantation of the elements and to provide or process the relevant characteristics for the elements in the implanted modelling position.

4. Device according to any one of claims 1 to 3, **characterised in that** the control means (7) comprise means which can be activated by foot or by voice in order to allow a surgeon to control the device without having to use his hands.

5. Device according to any one of claims 1 to 4, **characterised in that** the acquisition means which allow spatial data to be captured relating to the movements and to the relative positions of the femur and the tibia comprise a digital high-definition type camera (2) which is sensitive to signals from the bone components of the knee, such as, for example, infra-red reflection markers (4, 5) or, optionally, image recognition means.

## Patentansprüche

1. Vorrichtung zum Durchführen eines Verfahrens zur Selektion von einem (oder mehreren) Knieprothesenelement(en), und insbesondere von einem Oberschenkelprothesenteil und/oder Schienbeinkopfplateau-Prothesenteil innerhalb eines verfügbaren Satzes dieser Elemente, und/oder eines Oberschenkel- oder Schienbein-Dickenmaßes, wobei die genannte Vorrichtung umfasst:
eine Einrichtung (2, 3) zur Erfassung der räumlichen Position eines dreidimensionalen räumlichen Systems von Referenzmarkierungen (4, 5) auf dem Schienbein oder dem Oberschenkel eines Patienten, und der räumlichen Positionen von Markierungen, Tastern oder Instrumenten auf dem anderen Knochen, um räumliche Informationen bezüglich der Bewegung zwischen dem Oberschenkel und dem Schienbein eines Patienten in Höhe des Knies zu erhalten,
Einrichtungen, die vorzugsweise vorher erlangte anatomische Daten des Oberschenkels und/oder des Schienbeins des Patienten enthalten, um in Abhängigkeit von den genannten räumlichen Informationen bestimmen zu können: den Flexionswinkel zwischen dem Oberschenkel und dem Schienbein, den Abstand zwischen den Enden des Oberschenkels und dem Schienbein sowie den HKA-Winkel, das heißt den Innenwinkel, gebildet durch die mechanische Oberschenkelachse, definiert durch die Hüft-Mitte und die Knie-Mitte, und die mechanische Schienbeinachse, definiert durch die Knie-Mitte und die Knöchel-Mitte, Verarbeitungs- und Speichereinrichtungen (1) zur Speicherung des genannten Abstands und des genannten HKA-Winkels in Verbindung mit dem Flexionswinkel, für einen Extensionswinkel, insbesondere einen Winkel von 0° ± 5° und einen großen Flexionswinkel in der Größenordnung von 90°, insbesondere einen Winkel von 90° ± 15°,
Speichereinrichtungen, die Dimensionsinformationen bezüglich Knieimplantatskomponentensätzen enthalten,
3D-Verarbeitungseinrichtungen zur Modellierung der Implantationspositionen der genannten Elemente des Prothesenimplantatesatzes, wenigstens in den Positionen von Extensions- und großen Flexionswinkeln,
und Einrichtungen zur Verarbeitung, um relevante Informationen bezüglich des modellierten Knieimplantats zu liefern, und/oder zur Selektion der genannten Implantatesatzelemente, welche die besten charakteristischen Informationen liefern,
Einrichtungen (6) zur Anzeige der genannten Informationen oder Selektion,
und Steuereinrichtungen (7) für den Betrieb der Vorrichtung,
**dadurch gekennzeichnet, dass** die Verarbeitungs- und Speichereinrichtungen angepasst sind, außerdem den genannten Abstand und den genannten HKA-Winkel für wenigstens einen reduzierten Flexionswinkel zu speichern, insbesondere einen Winkel von 20° ± 10°, und **dadurch**, dass sie Einrichtungen zum Vergleichen der genannten Maße der HKA-Winkel wenigstens für den reduzierten Flexionswinkel und für den Extensionswinkel umfasst, angepasst um anzugeben ob, in diesen beiden Positionen, im Falle von Genu varum und im Falle von Genu valgum die HKA-Winkel im Wesentlichen gleich und zwischen tolerierbaren Grenzwerten enthalten sind.

2. Vorrichtung nach Anspruch 1, **dadurch gekennzeichnet, dass** die genannten Steuereinrichtungen (7) so konzipiert sind, dass sie dem Operator das Speichern der erfassten, wenigstens den genannten Winkeln der schwachen Flexion, der Extension und der starken Flexion entsprechenden Daten ermöglicht, wobei die genannten Verarbeitungseinrichtungen verifizieren und validieren, dass die Winkel, für welche die Daten erfasst werden, sehr wohl den vorerwähnten Winkeln entsprechen.

3. Vorrichtung nach einem der Ansprüche 1 und 2, **dadurch gekennzeichnet, dass** die Steuereinrichtungen (7) so konzipiert sind, dass sie dem Operator vorschlagen, ein oder mehrere Elemente des Prothesenelementesatzes zu wählen, um den genannten Verarbeitungseinrichtungen (1) zu ermöglichen, die Implantation der genannten Elemente zu modellieren und die relevanten Charakteristika für die genannten Elemente in Implantationsmodellierungsposition zu liefern.

4. Vorrichtung nach einem der Ansprüche 1 bis 3, **dadurch gekennzeichnet, dass** die Steuereinrichtungen (7) mit dem Fuß oder mit der Stimme betätigbare Einrichtungen umfassen, um einem Chirurgen zu ermöglichen, die Vorrichtung zu steuern, ohne seine Hände benutzen zu müssen.

5. Vorrichtung nach einem der Ansprüche 1 bis 4, **dadurch gekennzeichnet, dass** die Erfassungseinrichtungen, die ermöglichen, die räumlichen Informationen zu erfassen, welche die Bewegungen und die relativen Positionen des Oberschenkels und des Schienbeins betreffen, eine HD-Digitalkamera (2), empfindlich für von Knochenteilen des Knies stammende Signale, wie zum Beispiel von Infrarot-Reflexionsmarkierungen (4, 5), oder eventuell Bilderkennungseinrichtungen umfassen.
